# EUROPÄISCHE PATENTSCHRIFT

(11) **EP 0 572 751 B1**
(45) Veröffentlichungstag und Bekanntmachung des Hinweises auf die Patenterteilung: **27.12.1996**
(21) Anmeldenummer: 92810432.2
(22) Anmeldetag: 04.06.1992
(51) Int. Cl.: A61F 2/02

(54) **Endoprothese aus kompaktem, thermoplastischem Verbundwerkstoff**
Endoprosthesis of a dense thermoplastic compound
Endoprothèse en matériau composite thermoplastique compact

(43) Veröffentlichungstag der Anmeldung: 08.12.1993
(73) Patentinhaber: Sulzer Orthopädie AG, 6340 Baar (CH)
(72) Erfinder: Gysin, Hansjörg, CH-8400 Winterthur (CH); Streicher, Robert Michael, CH-8405 Winterthur (CH)
(74) Vertreter: Heubeck, Bernhard

(56) Entgegenhaltungen:
- DE-A- 3 613 657
- GB-A- 2 216 425
- US-A- 3 893 196
- US-A- 4 902 297

## Beschreibung

Die Erfindung betrifft eine Endoprothese aus kompaktem thermoplastischem Verbundwerkstoff mit technischen Endlosfasern mit einer Vorzugsrichtung. Solche Prothesen sind z.B. aus der WO 87/04916 bekannt. Deren Aufbau mit einem inneren unidirektionalen Kern und mehreren darüberliegenden Schichten ist jedoch sehr aufwendig und kompliziert herzustellen. Zudem können gefährliche mechanische Schwachstellen auftreten und die Zeitstandfestigkeit wird entsprechend stark eingeschränkt.

Es ist nun Aufgabe der vorliegenden Erfindung, eine Endoprothese mit einem Strukturaufbau zu schaffen, welche einfach und sicher herstellbar ist und welche durchgehend hohe spezifische mechanische Festigkeiten bezüglich Zug, Biegung und Torsion, sowie eine sehr gute Zeitstandfestigkeit aufweist. Vor allem sollen auch mechanische Schwachstellen vermieden werden. Diese Aufgabe erfüllt eine erfindungsgemässe Endoprothese nach Anspruch 1. Die in sich stabilen Langelemente, deren innige Verbindung miteinander und deren Ausrichtung entlang der Vorzugsrichtungen, ergeben Strukturen mit besonders guten spezifischen Festigkeitswerten bezüglich Zug-, Druck- und Torsion. Ueberdies werden damit lokale Schwachstellen auch in komplizierten Formen weitgehend vermieden.

Die abhängigen Ansprüche betreffen vorteilhafte Weiterbildungen der erfinderischen Strukturen. So können die Langelemente einen unidirektionalen Kern oder einen Kern aus einem langezogenen Geflecht mit kleinem Faserwinkel aufweisen. Der umhüllende Mantel kann auf einfache Art aus sich kreuzenden Gelegen oder aus einem Geflecht gebildet sein. Besonders gute mechanische Eigenschaften können mit hochkompaktierten Funktionsteilen mit einem Porenanteil von höchstens 1 % erreicht werden. Besonders geeignete Thermoplaste können sein: Polyacryle, Polyaryletherketone, Polycarbonate, Polyethersulfone, Polyethylene oder Polypropylene. Geeignete Endlosfasern können aus Kohle oder Aramid bestehen. Abgestimmt auf die Form der Prothese können besonders gute mechanische Eigenschaften auch erreicht werden: mit Langelementen, deren Länge mindestens das Fünffache ihres kleinsten Durchmessers beträgt oder mit Langelementen, deren Enden schräg auslaufen und so eine intensivere Verbindung zu benachbarten Langelementen ergeben. Die Langelemente können auch in ganzer Länge durch die Prothese durchgehend sein, d.h. nur an deren Oberflächen enden. Mit Vorteil sind auch in flachen Stellen der Prothese alle Langelemente mindestens teilweise durch ein benachbartes Langelement überdeckt und es können mindestens 6 Langelemente in einem Hauptquerschnitt liegen. Die erfindungsgemässen Funktionsteile können verschiedene Formen aufweisen: sie können an einem Ende auslaufend oder abgeflacht sein, und sie können schaufelförmige, sphärische oder schalenförmige Bereiche aufweisen. Mit dem erfindungsgemässen Strukturaufbau können vor allem auch Prothesen gebildet werden, welche hohen mechanischen Belastungen ausgesetzt sind. Besonders stabile Endoprothesen können einen Schaft zum Implantieren in einen Röhrenknochen aufweisen oder sie können einen Schaft bilden, welcher in eine Schale oder in einen gerundeten Kopf ausläuft. Durch thermoplastischen Einbau eines Metallgitters an der Oberfläche und durch Verankerung in derselben kann eine besonders gute Verbindung zum umgebenden Knochenmaterial oder dem Knochenzement erzielt werden. Es können auch Röntgenmarken an definierten Stellen in die Verbundwerkstoffprothese eingebaut werden zwecks postoperativer Kontrolle der Prothesenlage bezüglich des Knochens.

Die Erfindung wird im folgenden anhand von Beispielen und Figuren weiter erläutert. Es zeigen:
- Fig.1a,b,c: Eine erfindungsgemässe Endoprothese aus kompaktierten Langelementen und den Aufbau der Langelemente,
- Fig.2, 3: Beispiele von Langelementen mit innerem Kern und Mantel,
- Fig.4: ein Langelement mit schrägen Enden,
- Fig.5: eine Prothese mit durchgehenden Langelementen,
- Fig.6: einen Querschnitt durch eine Prothese mit verformten und sich überlappenden Langelementen,
- Fig.7: eine Endoprothese in einer Fliesspressform,
- Fig.8: eine Prothese mit einem Schaft in einem Röhrenknochen,
- Fig.9: einen gerundeten Kopf einer Prothese

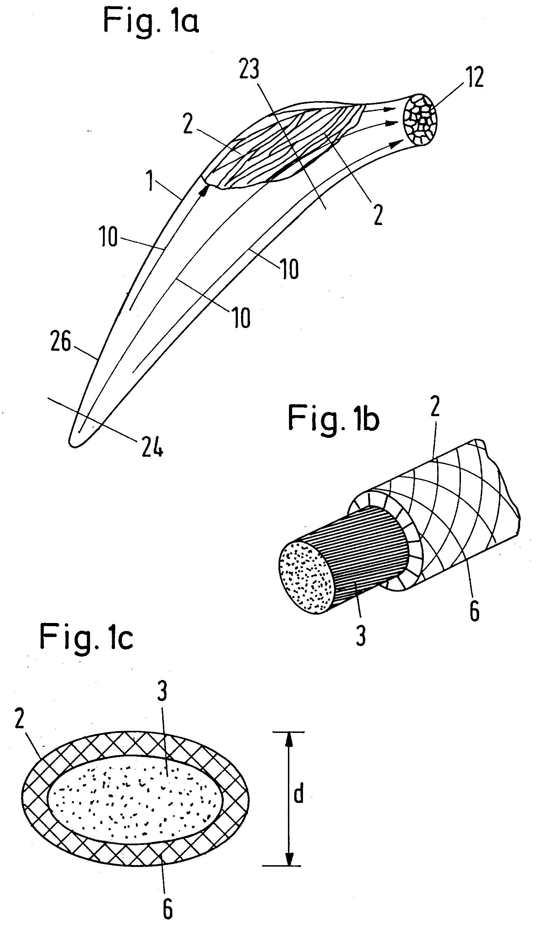

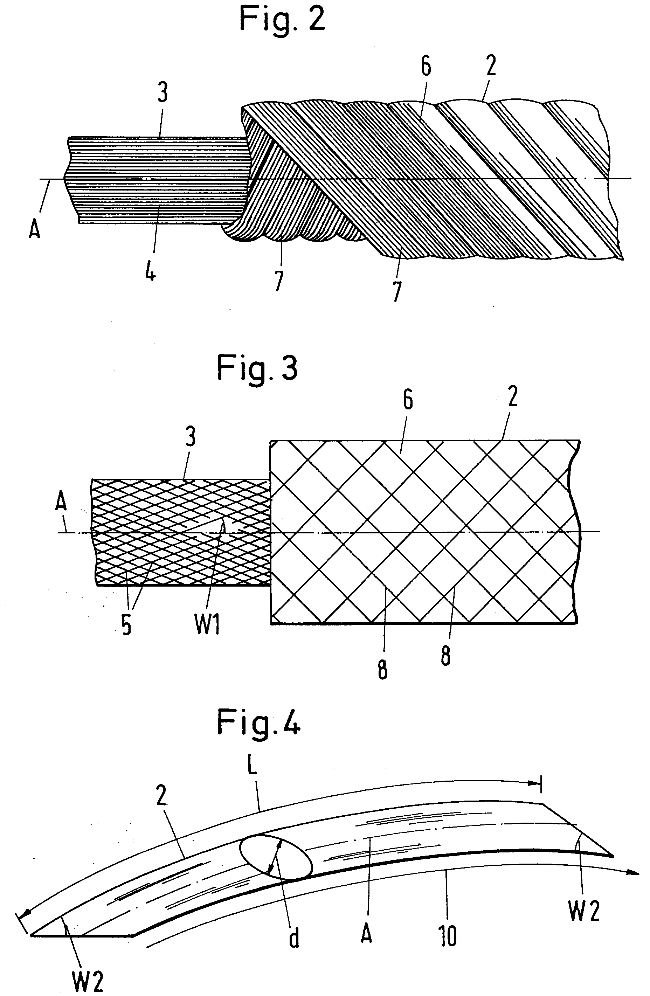

Figur 1a zeigt eine Endoprothese mit einem sich verjüngenden Ende 26. Die Prothese ist aus kompaktierten Langelementen 2 aufgebaut, welche an jeder Stelle entlang einer Vorzugsrichtung 10 orientiert und über ihre ganze Länge thermoplastisch miteinander verbunden sind. Die Vorzugsrichtungen 10 verlaufen hier ausgehend von einer Endfläche 12 bis zum Ende 26. Die Querschnittsform und -grösse verändert sich in Vorzugsrichtung 10 bzw. über das Funktionsteil 1 hinweg von einem kleinen Querschnitt 24 am verjüngten Ende 26 zu einem grösseren veränderten Querschnitt 23 im oberen Bereich der Prothese. Die Langelemente 2, in Fig.1b und im Querschnitt gezeigt in Fig.1c, weisen einen inneren Kern 3 mit grossteils längsorientierten Endlosfasern und einem umgebenden Mantel 6 mit sich kreuzenden Fasern auf. Figur 1c zeigt einen ovalen Querschnitt mit einem kleinsten Durchmesser d. Das Langelement 2 im Beispiel von Figur 2 weist einen unidirektionalen Kern 4 umgeben von zwei sich kreuzenden, übereinanderliegenden Gelegen 7 auf. In Figur 3 besteht der Kern aus einem langgezogenen Geflecht 5 mit einem Faserwinkel W1 von maximal 20° zur Elementachse A. Der umhüllende Mantel 6 besteht aus einem Geflecht 8 mit relativ grossem Faserwinkel von z.B. 35° - 45°. Beim Gelege 7 kann dieser Faserwinkel auch noch grösser sein, z.B. 45° - 55°. Durch den erfindungsgemässen Aufbau aus Langelementen 2 mit längsorientiertem Kern und stark gekreuzten Mantelfasern können auf einfache Art Prothesen mit in weiten Bereichen beliebigen Formen und mit ausgezeichneten mechanischen Festigkeits- und Torsionseigenschaften über das ganze Volumen der Prothese und ohne Schwachstellen hergestellt werden. Vermeidung von Schwachstellen ist bei Endoprothesen aus Verbundwerkstoffen besonders wichtig und auch entsprechend schwierig zu erreichen.

In Figur 4 ist ein Langelement 2 gezeigt, dessen Länge L mehr als das fünffache seines kleinsten Durchmessers d beträgt. Mit Vorteil meist sogar das zehnfache oder mehr. Die Enden des Langelements 2 sind schräg abgeschnitten bzw. laufen schräg aus mit einem Winkel W2 von z.B. 30° - 60° zur Elementachse A, wodurch die thermoplastische Verbindung zwischen verschiedenen Langelementen in der Prothese und deren Kompaktheit weiter verbessert werden kann.

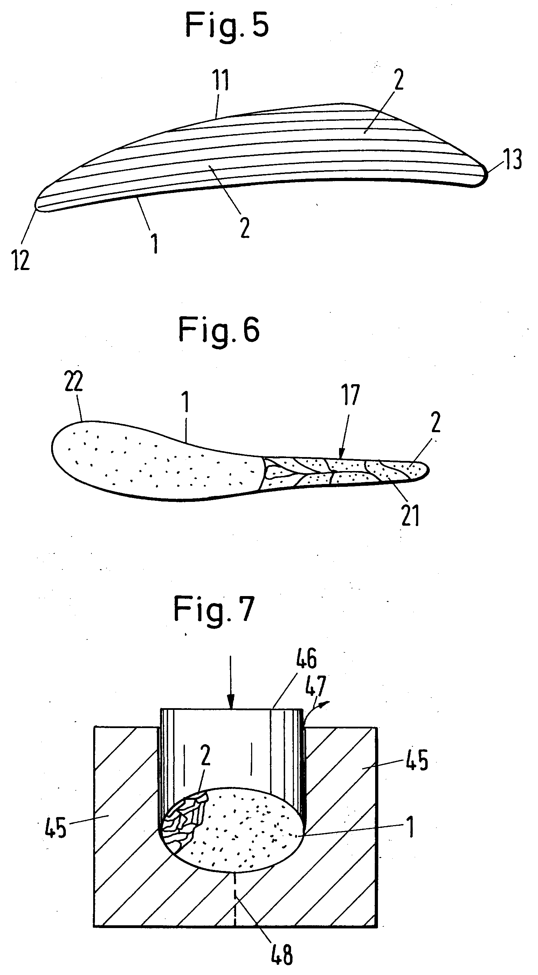

Strukturen mit besonders hoher Festigkeit können nach Figur 5 erreicht werden, indem alle Langelemente 2 an der Oberfläche 11 und vor allem an den Endflächen 12, 13 einer Prothese enden, d.h. wenn sie im Inneren durchgehend sind. Figur 6 zeigt einen Hauptquerschnitt durch eine Prothese mit einem abgeflachten Bereich 17, wobei auch hier immer noch jedes Langelement 2 mindestens teilweise durch ein benachbartes Langelement 21 überdeckt ist, um damit gute Festigkeitswerte sicherzustellen. In einem Hauptquerschnitt 22 sollten mit Vorteil mindestens sechs Langelemente 2 liegen.

Figur 7 zeigt eine Fliesspressform 45 mit einem Stempel 46 zur Herstellung erfindungsgemässer Endoprothese. Dabei werden Langelemente 2 in Vorzugsrichtung 10 und in der Prothese entsprechender Menge und Verteilung in die Form 45 heiss eingelegt, dann in der Form unter hohem Druck (z.B. mehrere 100 bar) und bei einer Temperatur über dem Erweichungspunkt durch Fliesspressen kompaktiert und dabei auch die Luft zwischen den Langelementen 2 ausgepresst (47). Anschliessend wird das Pressteil 1 unter Druck bis zur Erreichung der Formstabilität abgekühlt. Auf diese Art wird ein Porenanteil von weniger als 2 %, vorzugsweise von weniger als 1 %, erreicht. Die Pressform 45 kann entsprechend dem gewünschten Fliesspressteil 1 auch mehrteilig, z.B. in der Ebene 48 geteilt sein.

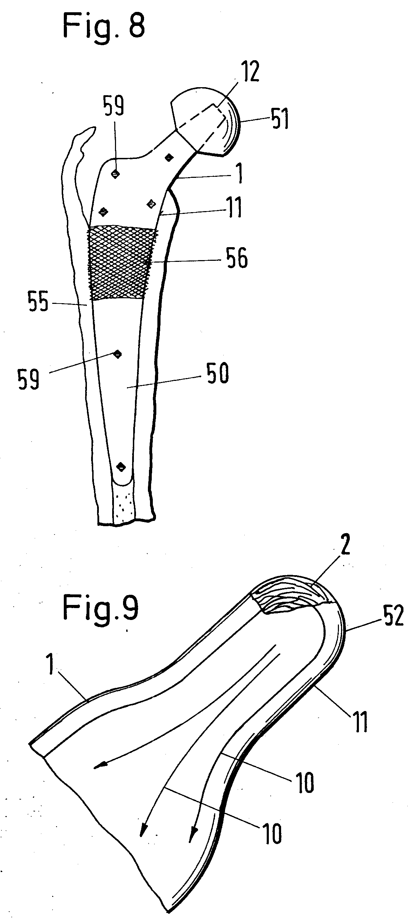

Der erfindungsgemässe Aufbau der Endoprothese ermöglicht es, eine grosse Vielfalt von Formen zu realisieren, wie dies durch die Beispiele illustriert wird. Figur 8 zeigt ein Beispiel einer Endoprothese mit einem Schaft 50, welcher in einen Röhrenknochen 55 implantiert ist. Im oberen Teil des Schafts 50 ist ein Metallgitter 56 z.B. ein Titangitter Sulmesh thermoplastisch in der Oberfläche 11 des Verbundwerkstoffschafts verankert. Dies ermöglicht eine dauerhafte und starke Verbindung von Knochen und Prothese infolge Einwachsens von Knochensubstanz ins Metallgitter 56. Am oberen Ende 12 ist eine Gelenkkugel 51 z.B. aus Metall oder Keramik aufgesetzt und mit der Prothese 1 verbunden. An definierten Stellen sind Röntgenmarken 59 in den Schaft 50 eingebaut. Dies erlaubt es, die relative Position der Prothese zum Knochen 55 auch nach der Operation genau zu bestimmen bzw. zu überwachen. Figur 9 zeigt ein Beispiel mit einem gerundeten Kopf 52 einer Endoprothese. Die Vorzugsrichtungen 10 und damit die Langelemente 2 verlaufen hier tangential zur Oberfläche 11 des Kopfs. Diese ergibt eine besonders stabile Oberfläche.

## Patentansprüche

1. Endoprothese aus kompaktem, thermoplastischem Verbundwerkstoff mit technischen Endlosfasern, mit einer Vorzugsrichtung (10), dadurch gekennzeichnet, dass die Prothese mehrere Langelemente (2) aufweist, welche im wesentlichen in Vorzugsrichtung (10) orientiert und über ihre ganze Länge thermoplastisch miteinander verbunden sind, wobei die Langelemente je aus einem inneren Kern (3) mit grossteils längsorientierten Fasern und einem umhüllenden Mantel (6) mit sich kreuzenden Fasern bestehen.

2. Endoprothese nach Anspruch 1, dadurch gekennzeichnet, dass die Langelemente einen unidirektionalen Kern (4) aufweisen.

3. Endoprothese nach Anspruch 1, dadurch gekennzeichnet, dass der innere Kern der Langelemente aus einem langgezogenen Geflecht (5) besteht mit einem Faserwinkel W1 von höchstens 20° zur Elementachse A.

4. Endoprothese nach einem der vorangehenden Ansprüche, dadurch gekennzeichnet, dass der umhüllende Mantel (6) aus sich kreuzenden Gelegen (7) oder aus einem Geflecht (8) besteht.

5. Endoprothese nach einem der vorangehenden Ansprüche, dadurch gekennzeichnet, dass der Porenanteil weniger als 1 % beträgt.

6. Endoprothese nach einem der vorangehenden Ansprüche, dadurch gekennzeichnet, dass als Thermoplaste Polyacryle, Polyaryletherketone, Polycarbonate, Polyethersulfone, Polyethylene oder Polypropylene vorgesehen sind.

7. Endoprothese nach einem der vorangehenden Ansprüche, gekennzeichnet durch Endlosfasern aus Kohle oder Aramid.

8. Endoprothese nach einem der vorangehenden Ansprüche, dadurch gekennzeichnet, dass die Länge L der Langelemente (2) mindestens das fünffache ihres kleinsten Durchmessers d beträgt.

9. Endoprothese nach einem der vorangehenden Ansprüche, dadurch gekennzeichent, dass die Langelemente (2) an ihren Enden schräg auslaufen mit einem Winkel W2 zwischen 30° und 60°.

10. Endoprothese nach einem der vorangehenden Ansprüche, dadurch gekennzeichnet, dass alle Langelemente (2) an deren Oberfläche (11), hauptsächlich an deren Endflächen (12,13) enden.

11. Endoprothese nach einem der vorangehenden Ansprüche, dadurch gekennzeichnet, dass alle Längselemente thermisch verformt sind und dass auch in flachen Querschnitten (17) zur Vorzugsrichtung jedes Langelement (2) mindestens teilweise durch ein benachbartes Langelement (21) überdeckt ist.

12. Endoprothese nach einem der vorangehenden Ansprüche, dadurch gekennzeichnet, dass sie an mindestens einem Ende sich verjüngend (26) ausgebildet ist.

13. Endoprothese nach einem der vorangehenden Ansprüche, gekennzeichnet durch einen Schaft (50) zum Implantieren in einen Röhrenknochen (55).

14. Endoprothese nach einem der vorangehenden Ansprüche, gekennzeichnet durch einen Schaft, welcher in eine Schale oder in einen gerundeten Kopf (52) ausläuft.

15. Endoprothese nach einem der vorangehenden Ansprüche, dadurch gekennzeichnet, dass ein Metallgitter (56) thermoplastisch in deren Oberfläche (11) verankert ist.

16. Endoprothese nach einem der vorangehenden Ansprüche, gekennzeichnet durch Röntgenmarken (59), welche an definierten Stellen eingebaut sind.

## Claims

1. An endoprosthesis of compact thermoplastics composite material with commercial endless fibres, having a preferred direction (10), characterised in that the prosthesis has a plurality of elongated elements (2) which are oriented substantially in the preferred direction (10) and are mutually connected thermoplastically over their entire length, the elongated elements each comprising an internal core (3) with predominantly longitudinally oriented fibres and an enclosing covering (6) with mutually crossing fibres.

2. An endoprosthesis as claimed in claim 1, characterised in that the elongated elements have a unidirectional core (4).

3. An endoprosthesis as claimed in claim 1, characterised in that the internal core of the elongated elements comprises an elongated braid (5) with a fibre angle W1 of at most 20° to the element axis A.

4. An endoprosthesis as claimed in any of the preceding claims, characterised in that the enclosing covering (6) comprises mutually crossing layers (7) or a braid (8).

5. An endoprosthesis as claimed in any of the preceding claims, characterised in that the proportion of voids is less than 1%.

6. An endoprosthesis as claimed in any of the preceding claims, characterised in that the thermoplastics provided are polyacrylates, polyaryl ether ketones, polycarbonates, polyether sulphones, polyethylenes or polypropylenes.

7. An endoprosthesis as claimed in any of the preceding claims, characterised by endless fibres of carbon or aramid.

8. An endoprosthesis as claimed in any of the preceding claims, characterised in that the length (L) of the elongated elements (2) is at least five times their minimum diameter (d).

9. An endoprosthesis as claimed in any of the preceding claims, characterised in that the elongated elements (2) finish obliquely at their ends with an angle W2 between 30° and 60°.

10. An endoprosthesis as claimed in any of the preceding claims, characterised in that all the elongated elements (2) end at its surface (11), and mainly at its end surfaces (12, 13).

11. An endoprosthesis as claimed in any of the preceding claims, characterised in that all the elongated elements are thermally shaped, and even in shallow cross-sections (17) relative to the preferred direction each elongated element (2) is at least partly overlapped by an adjoining elongated element (21).

12. An endoprosthesis as claimed in any of the preceding claims, characterised in that it tapers (26) at at least one end.

13. An endoprosthesis as claimed in any of the preceding claims, characterised by a stem (50) for implantation in a tubular bone (55).

14. An endoprosthesis as claimed in any of the preceding claims, characterised by a stem which ends in a cup or a rounded head (52).

15. An endoprosthesis as claimed in any of the preceding claims, characterised in that a metal mesh (56) is thermoplastically anchored in the prosthesis surface (11).

16. An endoprosthesis as claimed in any of the preceding claims, characterised by radiography markers (59) built in at predetermined places.

## Revendications

1. Endoprothèse en matériau composite thermoplastique compact, comportant des fibres continues techniques, avec une orientation préférentielle (10), caractérisée en ce que la prothèse présente plusieurs éléments allongés (2), orientés essentiellement selon une direction préférentielle (10) et reliés ensemble de façon thermoplastique sur toute leur longueur, les éléments allongés étant chacun constitué d'un noyau interne (3) avec des fibres orientées longitudinalement pour leur plus grande part et une enveloppe (6) d'enrobage comportant des fibres se croisant.

2. Endoprothèse selon la revendication 1, caractérisée en ce que les éléments allongés présentent un noyau (4) unidirectionnel.

3. Endoprothèse selon la revendication 1, caractérisée en ce que le noyau intérieur des éléments allongés est constitué d'un treillis (5) étiré longitudinalement, ayant un angle de fibre W1 maximal de 20° par rapport à l'axe d'élément A.

4. Endoprothèse selon l'une des revendications précédentes, caractérisée en ce que l'enveloppe (6) d'enrobage est constituée de nappes (7) se croisant ou d'un treillis (8).

5. Endoprothèse selon l'une des revendications précédentes, caractérisée en ce que la proportion de pores est inférieure à 1 %.

6. Endoprothèse selon l'une des revendications précédentes, caractérisée en ce que sont prévus comme thermoplastiques des polyacryles, polyaryléthercétones, polycarbonates, polyéthersulfones, polyéthylènes ou polypropylènes.

7. Endoprothèse selon l'une des revendications précédentes, caractérisée par des fibres continues, en carbone ou en aramide.

8. Endoprothèse selon l'une des revendications précédentes, caractérisée en ce que la longueur L des éléments allongés (2) est au moins du quintuple de leur diamètre minimal d.

9. Endoprothèse selon l'une des revendications précédentes, caractérisée en ce que les éléments allongés (2) ont une allure oblique à leurs extrémités, selon un angle W2 situé dans la plage comprise entre 30 et 60°.

10. Endoprothèse selon l'une des revendications précédentes, caractérisée en ce que tous les éléments allongés (2) s'achèvent sur sa surface (11), principalement en ses surfaces d'extrémité (12, 13).

11. Endoprothèse selon l'une des revendications précédentes, caractérisée en ce que tous les éléments allongés sont conformés par voie thermique et en ce que, également dans des sections transversales (17) plates par rapport à la direction préférentielle, chaque élément (2) est au moins partiellement recouvert par un élément allongé (21) voisin.

12. Endoprothèse selon l'une des revendications précédentes, caractérisée en ce qu'elle est réalisée à au moins une extrémité, avec un effilement (26).

13. Endoprothèse selon l'une des revendications précédentes, caractérisée par une tige (50) destinée à l'implantation dans un os tubulaire (55).

14. Endoprothèse selon l'une des revendications précédentes, caractérisée par une tige, qui continue en formant une cupule ou une tête arrondie (52).

15. Endoprothèse selon l'une des revendications précédentes, caractérisée en ce qu'une grille métallique (56) est ancrée, par voie thermoplastique, dans sa surface (11).

16. Endoprothèse selon l'une des revendications précédentes, caractérisé par des marques pour rayons X (59), intégrées en des endroits définis.
